# EUROPEAN PATENT SPECIFICATION

(11) **EP 4 366 619 B1**
(45) Date of publication and mention of the grant of the patent: **03.06.2026**
(21) Application number: 22738636.4
(22) Date of filing: 28.06.2022
(51) Int. Cl.: A61B 5/363, A61B 5/367, G16H 20/40, G16H 50/20, A61B 5/00, A61B 18/14

(54) **ATRIAL TACHYCARDIA ANALYSIS MODULE PROVIDING EVOLVING BLUEPRINT OF TACHYCARDIA EVENTS**
MODUL ZUR ANALYSE VON VORHOFTACHYKARDIE MIT SICH ENTWICKELNDEM BLAUDRUCK VON TACHYKARDIEEREIGNISSEN
MODULE D'ANALYSE DE TACHYCARDIE AURICULAIRE FOURNISSANT UN PLAN ÉVOLUTIF D'ÉVÉNEMENTS DE TACHYCARDIE

(30) Priority: 09.07.2021 FR 2107470
(43) Date of publication of application: 15.05.2024
(73) Proprietor: Substrate HD, 13006 Marseille (FR)
(72) Inventor: VICTORINO CARDOSO, Gabriel, 13006 Marseille (FR); BOUDOU, Thomas, 13006 Marseille (FR)
(74) Representative: Plasseraud IP
(86) International application number: PCT/EP2022/067814
(87) International publication number: WO 2023/280645

(56) References cited:
- US-A1- 2014 243 641
- US-A1- 2020 245 885
- ALHUSSEINI MAHMOOD I. ET AL: "Machine Learning to Classify Intracardiac Electrical Patterns During Atrial Fibrillation : Machine Learning of Atrial Fibrillation", CIRCULATION: ARRHYTHMIA AND ELECTROPHYSIOLOGY, vol. 13, no. 8, 6 July 2020 (2020-07-06), United States, XP055862043, ISSN: 1941-3149, DOI: 10.1161/CIRCEP.119.008160
- TRAYANOVA NATALIA A. ET AL: "Machine Learning in Arrhythmia and Electrophysiology", CIRCULATION RESEARCH, vol. 128, no. 4, 19 February 2021 (2021-02-19), US, pages 544 - 566, XP055904166, ISSN: 0009-7330, DOI: 10.1161/CIRCRESAHA.120.317872

## Description

The present disclosure is directed to systems and methods for data-driven, continuous, and adaptable learning approaches for analyzing atrial tachycardia (AT) in a patient.

These approaches can be used during a catheter ablation procedure to provide information to a medical professional during the ablation procedure regarding the AT. The human heart has four chambers: the left and right atria (atrial chambers) and the left and right ventricles (ventricular chambers). During normal operation, blood flows into the right atrium and is pumped through the right ventricle into the lungs, where the blood is oxygenated. The blood then returns from the lungs into the left atrium and is pumped through the left ventricle, where the oxygenated blood is distributed throughout the body. An electrical signal contracts the heart muscles, which allows the heart to pump blood through the circulatory system. The pulmonary circuits are the paths by which blood travels from the heart to the lungs and back to the heart. The systemic circuits are the paths by which blood travels from the heart to the body and back to the heart.

Atrial tachycardia (AT) is an electrical abnormality in which there is a faster than normal activity in the atrial chambers of the heart. The activity during an AT is not coordinated by the sinusal node, which is the part of the atrium that normally coordinates the heart's electrical signals. Instead, the activity is coordinated by a certain region of the atrium that is either maintaining an electrical feedback loop or is spontaneously emitting electrical signals. An electrical feedback loop in the heart is commonly called "reentry mechanism".

During an AT, the electrical signals disturb the rhythm of the normal atrial contractions. This can impact the patient's health and even threaten the patient's life. Symptoms of AT may include chest palpitations, fainting, dizziness, sweating, chest pain, shortness of breath, fatigue, weakness, and even heart failure. Therefore, doctors aim to treat AT events in patients to restore normal atrial contractions. Electrophysiologists (EP) are typically the doctors that treat AT, especially where surgery is concerned. However, other doctors can also treat AT. The use of the term EP in this disclosure may therefore refer to both electrophysiologists and other doctors.

One of the procedures used to treat AT is catheter ablation of the region that is the source of the abnormal electrical signal. In a typical AT ablation procedure, the EP sedates the patient and inserts two catheters into the patient's heart through the patient's veins. An incision is made to access the patient's veins, typically in the upper part of the patient's right leg. One of the catheters is the coronary sinus catheter, which is a reference catheter that is placed in a vein inside the coronary sinus. This vein is important because it travels around the left atrium and it therefore can be a good reference for the recording of the patient's atrial electrical activity. The second catheter is the catheter that the EP will move inside the atrium during the ablation procedure to investigate the electrical signals. During the procedure, the EP attempts to locate the region that is responsible for causing the AT. Such region may either be a region containing a focus from which pathological atrial activation spreads centrifugally, or the circuit of the reentry mechanism. If the patient is not experiencing an AT, the EP will try to trigger AT via electrical stimulation. Once the AT is triggered, the EP will try to investigate the electrical signals to detect the region that is causing the AT.

An EP can locate the responsible region in at least two ways. First, the EP can use the electrical signals of the heart. According to this method, the EP tries to find electrical signals in the middle of the atrial diastole. The atrial diastole is the time interval between two consecutive contractions of the atria. A contraction of the atrium is caused by the propagation of an electrical signal through the atrium. A typical atrium beat lasts for about 50 microseconds, and the time between atrium beats is about 600 to 1000 microseconds. An electrical signal in the middle of the atrial diastole is likely to be associated to an AT, as it separated in time from the electrical signals that caused the contractions of the atria. If the EP finds a region that is active in the middle of the time period between atrium contractions, this region is more likely to be causing an AT. Software can be used to help an EP find the middle of the atrial diastoles by visualizing the electrical signals of the heart. This software can visually show electrical characteristics over a period of time. For example, the voltage and current in a region can be shown to the EP over a period of 10 seconds. As the contractions of the atria typically create an electrical signal pattern, this visualization can help the EP find the region causing the AT. The displays available to the EP to see the electrical signals of the patient's heart can be transmitted from the EP recording system, which is typically a mobile cart or workstation that has the necessary hardware connections to receive signals during the ablation procedure.

Second, the EP can use a 3D-mapping system that builds a visual map of the electrical signals propagating throughout the heart. The EP creates this 3D map by taking measurements of the heart with a catheter. The amount of measurements needed varies with the 3D-mapping system, the desired precision, and the time available to the EP to perform the ablation surgery. If this 3D map is created successfully, the EP can use the 3D map to analyze the electrical signals in order to identify the region that is propagating the AT.

Once the EP identifies the region that is propagating the AT, the EP ablates the region or performs a transection of the circuit in the case of reentry mechanism of AT with the aim to stop the AT and reestablish normal sinus rhythm in the heart.

However, while the EP is performing the ablation procedure, another region may spontaneously take over for the previous region and thus change the character of the AT. The character of the AT may also change during the procedure because another region in another part of the atria took over the targeted region while the EP is ablating the target region. Thus, it is important for an EP to have an understanding of a profile of the ongoing AT in order to determine if the previous information regarding the AT is still relevant, or if the AT has changed such that the information the EP is accessing concern a different AT event altogether. If the AT has changed significantly, the EP may need to reconsider the regions to be ablated, and rethink the ablation procedure. This reconsideration conventionally leads to cancelling the current visual map, and redo another one because the current visual map has become irrelevant and may even lead to false diagnosis.

There is therefore a need to provide an EP with an analysis and blueprint of an ongoing tachycardia which are automatically generated and updated. In particular, there is a need for a system and method for automatically providing an EP information regarding ongoing and changing AT events during a procedure, allowing an EP to easily determine whether an AT discovered in a new region is related to a previous AT, with minimal interaction from the EP or other medical professionals. Previous methods and systems are not easily adaptable for an EP, especially during surgery, as they are generally based on triggers and thresholds that must be manually input, such as the beat acceptance criteria in Boston Scientific's RHYTHMIA acquisition system.

The related prior art includes the following: US 2014/243641 A1 that relates to cardiac mapping technology, more specifically a real-time, direct visual mapping method and system for mapping and ablating cardiac arrhythmias; US 2020/245885 A1 that discloses methods for discovering with enhanced precision the locations of the sources of different types of cardiac rhythm disorders and irregularities in a patient's heart; ALHUSSEINI MAHMOOD I. ET AL: "Machine Learning to Classify Intracardiac Electrical Patterns During Atrial Fibrillation : Machine Learning of Atrial Fibrillation",CIRCULATION: ARRHYTHMIA AND ELECTROPHYSIOLOGY, vol. 13, no. 8, 6 July 2020 (2020-07-06), XP055862043, that discloses panoramic recording of bi-atrial electrical signals in AF; TRAYANOVA NATALIA A. ET AL: "Machine Learning in Arrhythmia and Electrophysiology",CIRCULATION RESEARCH, vol. 128, no. 4, 19 February 2021 (2021-02-19), pages 544-566, XP055904166, that discloses ML applications in cardiac electrophysiology and arrhythmias.

The invention is defined in the appended set of claims.

The invention aims at improving the situation. To this end, the disclosure is directed to systems and methods related to tracking, analyzing, and displaying information related to AT events in a patient. In particular, the system and methods are directed to receiving information regarding recordings of the electrical activity of the heart, analyzing the electrical activity recordings by comparing the electrical activity to a determined blueprint of the current AT event, and generating warnings if a detection is made that the AT has changed. A change in the character of the AT can be identified by a change in the electrical signal measured by instruments such as electrocardiogram (ECG) leads or coronary sinus catheter leads. Such a change indicates that the mechanism that is generating the electrical signal is different and is probably not located in the same region.

The blueprint of an AT is built in real-time based on the electrical signal measurements of the AT from various leads and includes several different features. The various leads can include, for example, a 12-lead ECG and/or coronary sinus catheter leads. Features can include, for example, the delay between the activations in the different leads, the profile of the electrical activations in the different leads, the profile of the electrical activations corresponding to the atrium in each lead, and the cycle length. The cycle length is the time period between two electrical waves, resulting from contractions of the atria. The blueprint is updated periodically by a training function. No prior data is needed, as all the necessary data is captured during the procedure through automatic signal analysis.

The system and method disclosed herein automatically create an evolving blueprint (or AT profile) of an ongoing AT such that a subsequent change in the AT is more accurately recognized and categorized. The system and method can use any or all data from the various leads. Typically, however, not all data collected during the procedure is used when updating or evaluating the blueprint. Instead, only the data collected most recently may be used as it is the closest in time to the current state of the patient. Various subsets of data can also be weighted more or less heavily. For example, the data may be weighted such that more recently acquired data is given more weight than older data. In this manner, the system and method can recognize changes in the AT profile and create an evolving blueprint. The system and method can then react quickly when something unusual happens, which might be used as a warning for the EP that something is fundamentally different with the mechanism behind the current AT. Because the AT can change during a procedure in real-time, it is important for the EP to understand that the previous data may no longer be relevant when the AT profile has changed during the ablation procedure. The EP can also use recorded information about the patient's previous AT profile in a current or subsequent procedure to determine whether a patient is experiencing a previously measured AT.

The system and method include and use a computer system configured to store heart electrical activity data, and a processor configured to implement a program to perform data analysis related to the received heart electrical activity data. As part of the data analysis, the computer system implements data-driven, continuous, and adaptable learning approaches to analyzing ATs in a patient. The system and method may generate an initial blueprint of the AT without human intervention and/or manual initial configuration of an AT profile. Moreover, the system and method track AT events, and adapt to provide an evolving AT profile without the need for human intervention. The system and method may obtain various input data from a patient, such as data from an ECG or coronary sinus catheter. For example, a conventional 12 lead-ECG may be used, with ten electrodes placed at different locations on the patient's body such that the magnitude of the heart's electrical potential is measured from twelve different angles over a data acquisition period. A coronary sinus catheter may be also used to sense electrical activity of the heart. The acquired data may then be processed, analyzed, and used to provide alerts to an EP. For example, to build the blueprint of the AT and compare received electrical activity to this blueprint, the system and method may implement an idle function, a training function, and a detection function.

The system and method allow an EP to more easily recognize when an AT has changed during a procedure and to more easily recognize when an AT matches a previously recorded AT. The system and method therefore provide for lower human mortality rates, both during or outside of surgery, easier and quicker analysis of ATs by EPs or other medical professionals, the ability to identify more complex AT configurations, increased success rates for medical procedures, a better quality of life for patients, and lower medical costs.

The disclosure is thus including a computer implemented method for analyzing an atrial tachycardia signal comprising the following operations:
a) collecting a set of data which represent recordings of electrical activity of a human heart from at least one input source;
b) creating a current atrial tachycardia profile using said set of data;
c) collecting a current data point of an atrial tachycardia signal from at least one input source; and
d) analyzing the current data point using the current atrial tachycardia profile in order to determine whether said current data point is an outlier with respect to said current atrial tachycardia profile.

No method is part of the invention as claimed.

In various embodiments, the method may comprise one or more of the following features:
- step b) comprises extracting features chosen in a group comprising a cycle length feature, an electrode activation sequence feature, and/or a morphological feature of the activation sequence, and training a feature-based anomaly detection algorithm using at least one of said extracted features and/or a combination thereof,
- step b) comprises augmenting the data of the extracted features prior to training said feature-based anomaly detection algorithm,
- step d) comprises extracting from said current data point the features which have been used to train said one class support vector machine, feeding said extracted features to said feature-based anomaly detection algorithm, and receiving in return a value indicating whether said current data point is an outlier with respect to said current atrial tachycardia profile,
- said feature-based anomaly detection algorithm is a one class support vector machine;
- the method further comprises : e) emitting an outlier warning upon determining that the current data point is an outlier, and repeating steps c) and d) with said current atrial tachycardia profile,
- the method further comprises f) analyzing the current data point using the current atrial tachycardia profile in order to determine whether the current data point is a persistent outlier; and g) emitting a change warning indicating a change in the current atrial tachycardia profile upon determining that the current data point is a persistent outlier,
- the method further comprises h) collecting a new set of data which represent recordings of electrical activity of a human heart from at least one input source; and i) creating a new current atrial tachycardia profile using the second set of data, and repeating steps c) and d) with said new current atrial tachycardia profile,
- when operation d) determines that the current data point is not an outlier the method further comprises d1) adding the current data point to a memory buffer,
- step d) further comprises d2) determining that a memory buffer threshold for data has been reached; and j) creating a new current atrial tachycardia profile using the data stored in the memory buffer and repeating operations c) and d) with said new current atrial tachycardia profile, and
- step d) further comprises d3) determining that a memory buffer threshold for data has not been reached, and repeating operations c) and d) with said current atrial tachycardia profile.

The disclosure also includes a computer program comprising instructions for performing the method according to the disclosure, a data storage medium having recorded thereon this computer program, and a computer system comprising a processor coupled to a memory having recorded thereon this computer program.

Other features and advantages of the disclosure will readily appear in the following description of the drawings, which show exemplary embodiments of the disclosure and on which:
[FIG. 1] illustrates a general diagram showing a system for implementing the AT analysis module according to the disclosure,
[FIG. 2] shows a general diagram showing functions performed by the AT analysis module of figure 1.
[FIG. 3] shows an exemplary diagram of the operations performed in the detection function of figure 2.

The drawings and the following description are comprised for the most part of positive and well-defined features.

The embodiments described herein are directed to analyzing ATs in a patient using input data regarding electrical activity of a patient's heart, hereinafter heart electrical activity data. The system and method implement a data-driven and continuous learning approach, whereby received heart electrical activity data is processed and compared to a blueprint of the AT in order to determine whether unexpected changes in the AT are occurring or have occurred.

First, the received heart electrical activity data may be used to build a profile or blueprint of the patient's heart electrical activity including information regarding existing ATs. Based on this, profiles and blueprints of existing ATs may be generated. Complex AT configurations can be identified using techniques such as density estimation and data augmentation. The distribution of certain features of the measured heart electrical activity data signals are used in density estimation techniques to define blueprint feature value ranges.

For example, the cycle length can be used (that is the time interval between two electric activations measured between two leads). If values are received at 200, 199, 201, and 202 milliseconds are measured for the cycle length, there may be a dense region around 200 milliseconds with a lower bound of around 199 milliseconds and an upper bound of around 202 milliseconds. Anything outside this range may be considered in a not dense region. This value range estimation process could be performed over a set of several features combined together.

An example of an algorithm that can be used to detect outliers in a data point cloud is a one class SVM (for support vector machine). The one class SVM is trained to learn a rough, close frontier delimiting the contour of the initial observations distribution. Further observations which lay within this frontier are thereafter considered as coming from the same population as the initial observations. On the opposite, if they lay outside the frontier, they are considered abnormal with a level of confidence which can be derived from the training. For instance, in the above example, a cycle length of 250 milliseconds would be well outside the range of 199 to 202 milliseconds.

Data augmentation techniques may be used to modify the input data to achieve a more robust and stable behavior of detection of the outliers . Examples of data augmentation techniques to improve AT detection include adding an expected noise or tolerance to measured data such that a change within this tolerance would not indicate a change in the AT, increasing the statistical significance of data points within a specified range, and categorizing data points that meet specific criteria as outliers such that they do not indicate a change in the AT. Alternatively, the SVM could be replaced by another frontier estimation technique. For example, such a frontier for a cycle length data point could be based on a tolerance of ±1 millisecond.

Using these and other techniques, outliers can be determined by comparing data points to previous data points. In other words, it can be determined whether the data point falls within the data point cloud of the previous data points. For example, a cycle length measurement of 203 milliseconds would not be detected as an outlier if the expected range was 199 to 202 milliseconds.

During an ablation procedure, heart electrical activity data is received, and processed to be compared to the current blueprint. Heart electrical activity data lying outside of an expected range can therefore be identified.

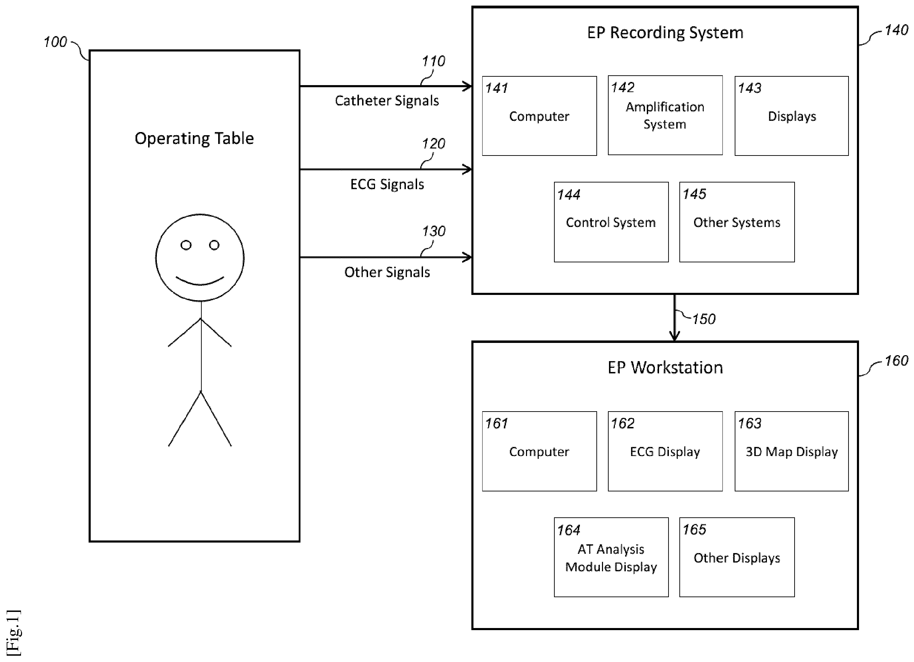

FIG. 1 illustrates a system implementing the AT analysis module according to the disclosure in an ablation procedure. In a typical procedure, the patient is on an operating table 100. Various leads and sensors, such as ECG leads and electrodes and a coronary sinus catheter, can be inserted into or attached to the patient while performing an ablation procedure or other procedure.

As described above, during ablation procedures, it is typical to insert catheter leads into the patient's heart and to attach ECG leads to the patient. The resulting catheter signals 110 and the ECG signals 120 are transmitted from the operating table 100 to an EP recording system 140. Numerous other signals 130 can also be transmitted from the operating table 100 to the EP recording system 140, such as signals including data regarding blood pressure, temperature, and blood oxygen level. Signals from the operating table 100 to the EP recording system 140 can be transmitted via a wired or wireless connection. The EP recording system 140 may be connected, via wired or wireless connections, to an EP workstation 160. The EP workstation may in the same room or area as the EP recording system, or may be in a location remote from the EP recording system.

The EP recording system 140 may be a mobile cart or workstation that has the necessary hardware connections to receive signals during the ablation procedure. The EP recording system may include a computer 141, amplification system 142, one or more displays 143, a control system 144, and other systems 145 related to monitoring the patient, guiding the EP during the procedure, or providing information to or receiving information from the EP.

Signals received by the EP recording system 140 can be amplified using amplification system 142 before being processed by the computer 141 for analysis. The computer 141 can comprise one computer or multiple computers, and may include a processor, memory, communication interfaces, and user input interfaces. For instance, the ECG signals 120 can be transmitted directly to a display 143 connected to a computer specifically for analyzing ECG signals, and another computer can analyze the remainder of the incoming signals.

After analysis of the incoming signals, computer 141 can display information on the EP recording system one or more displays 143 and/or transmit the information to the EP workstation 160 where it may be displayed on one or more displays at the EP workstation 160. Using the control system 144 of the EP recording system, the operator can control the computer 141, amplification system 142, and displays 143 of the EP recording system. The operator may also use the control system 144 to control a computer 161 and various displays at the EP workstation 160, or other systems related to the procedure.

For instance, the operator can change the displayed time period for the ECG signal from 10 seconds to 5 seconds on the ECG display 162 at the EP workstation or one of the displays 143 at the EP recording system. Various other systems 145 can also be implemented at the EP recording system 140 to assist the operator and/or EP in the procedure. For instance, the hospital paging system can be connected at the EP recording system 140.

The EP workstation 160 is where the EP performs the ablation procedure. The EP workstation 160 receives information from the EP recording system 140 and can have various displays depending on the preference of the EP. The transmission of information 150 between the EP recording system 140 and the EP workstation 160 can be via a wired or wireless connection, or a combination thereof. For example, the EP can use an ECG display 162 and/or a 3D map display 163 to help locate the region to be ablated.

Various other displays 165 can also be used to aid the EP. For instance, the patient's chart can be available on a display for the EP to reference and take notes during the procedure.

The computer 161 at the EP workstation can receive signals processed by the computer 141 at the EP recording system or unprocessed signals. Computer 161 further implements an AT analysis module according to the invention, and outputs results from the module to AT analysis module display 164. However, the computer 141 at the EP recording system can also be used to implement the AT analysis module. The AT analysis module may be a program module contained in a computer readable memory, that is then executed by a computer processor. The corresponding processing can be carried out using an instruction set implemented within a programmable computer that can include one or more non-transitory, tangible, computer readable storage media. For example, the programmable computer may include magnetic or optical storage media, solid-state electronic storage devices such as random access memory (RAM), or read-only memory (ROM), or any other physical device or media employed to store the instructions for carrying out the desired processing. The AT analysis module may include a memory for storing the received input data from a patient, or may be in communication with a memory containing such data. Further, a number of computers can be used to implement the AT analysis module, for instance a remote cloud system, so long as an output is made available to the EP in real-time or near real-time.

The computer systems 141 and 161 can each include at least one processor, memory, at least one storage device, and input/output devices. Some or all of the components can be interconnected via a system bus. The processor can be single- or multi-threaded and can have one or more cores. The processor can execute instructions, such as those stored in the memory and/or in the storage device. Information can be received and output using one or more I/O devices, including wired and wireless connections to other computer systems.

The above description is exemplary. Various other configurations are possible. For instance, the EP recording system 140 and EP workstation 160 could be combined or various functions of the EP recording system 140 and EP workstation 160 could be performed remotely in a cloud-based system.

The system and method include a computer system configured to receive heart electrical activity data. This data may be obtained, for example, from a 12-lead ECG, a coronary sinus catheter, or other devices that monitor electrical activity in a patient's heart. The recordings may be obtained by a computer used in the ablation procedure and then transmitted to a computer containing program modules for carrying out processing. The program modules generate all of the features from the recordings and implements the various phases described below.

From the analysis of the recordings, different features of the electrical activity of the patient's heart that are characteristic and stable under tachycardia can be determined. Features are considered stable under tachycardia when they are not changing to a specified tolerance during an AT with the same conditions. Such features are stable because the same region of the heart generates the electrical wave responsible for the activity in the atrium. The same electrical wave will have the same features measured and also the same features calculated based on the measured features. These features may include, for example, cardiac cycle lengths, p-wave morphology, activation profiles, coronary sinus activation sequences, other measurable data from an ECG or measurement device, and a number of features calculated from the measurable data. These features can be measured by, for example, ECG leads or coronary sinus catheter leads.

By analyzing the input data and comparing it to the AT profile, the system and method determines whether the received data is consistent with the profile it has made of the current AT, or if it is an outlier. The system may provide an output indication as to whether the received data is consistent with the AT profile, or whether it is an outlier. This indication may be provided in real-time, and can be visual, auditory, tactile, or any other method that would notify or alert the EP.

For example, a visual indicator could be a window on a computer display that turns red when it is normally white text on a black background. An auditory indicator could be a beep or a clicking sound. A tactile indictor could be a vibration.

If several outliers are detected in a row, the system transmits a warning that there is a change in the AT profile. This warning may be used by an EP or other medical professional during surgery or in other medical procedures to inform their decisions on how to best treat a patient.

Outliers can be detected in numerous ways in order to exclude data points that are not coherent with previous data. One method of determining outliers is to use a data point cloud in which the distance to the center of the data point cloud is measured. If the data point is inside the cloud, it is not an outlier. If it is outside the cloud, it is an outlier. For instance, the data point cloud may be centered upon a cycle length of 150 milliseconds. If a data point of 300 milliseconds was measured, it might be considered an outlier, whereas a data point of 160 milliseconds might not. Other methods of detecting outliers can also be used.

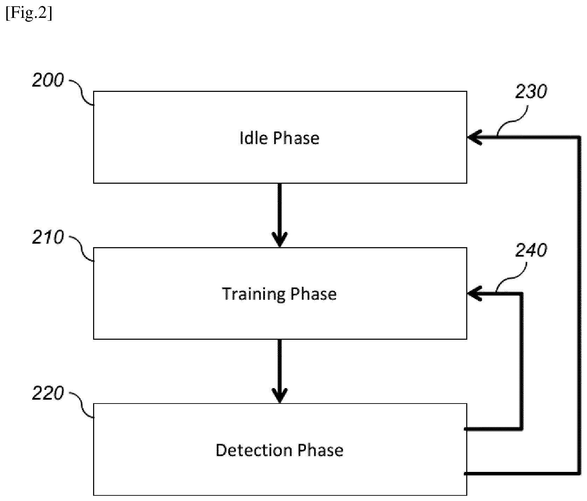

In certain embodiments, the heart electrical activity data is used by the AT analysis module. The AT analysis module may be implemented as a software program executed by a computer having the received heart electrical data stored in a local memory, or configured to access heart electrical activity data stored on a remote computer or server. As shown in FIG. 2, the AT analysis module executes an idle function which collects and stores data; a training function which uses received heart electrical activity data to generate a density estimation providing an AT profile; and a detection function where new, incoming data is compared to the AT profile to determine if the new incoming data is an outlier.

The idle function 200 of the AT analysis module collects and stores input data representing various features of the patient's heart, such as electrical activity data from an ECG or catheter probe. The EP may or may not be performing the ablation procedure during the idle phase, depending on his preference. The idle function 200 is typically executed for 10 to 15 seconds. Once a sufficient amount of input data is collected, the AT analysis module has enough input data to create an AT profile. In alternative embodiments, idle function 200 may collect input data for a defined period of time, until a predefined number of data points are collected, or until the AT analysis module determines that a profile being generated is of sufficient quality. An example of a predefined number of data points could be a specified number of p-waves. An operator may also manually instruct the analysis module when to begin and end collection of data. An operator may also manually instruct a separate module to collect data and automatically start the analysis module once certain conditions occur, such as measurements indicating a potential AT switch. The operator can further configure a memory buffer to hold a certain time period of data and execute the training function after a specific period of time. For example, the operator could configure the memory buffer to hold 3 minutes of data and execute the training function after 20 seconds. After the execution of the idle function 200, the AT analysis module executes the training function 210.

The training function 210 execution can be triggered after either the idle function 200 or the detection function 220. In the training function 210, the AT analysis module uses the most recent heart electrical activity data available to build the current blueprint. For example, a one class SVM can be used to receive several types of features which are defined for the AT profile. Such features may be based on at least a cycle length feature, an electrode activation sequence feature (that is a sequence of electrodes being successively activated), and/or a morphological feature of the activation sequence. Prior to the training of the one class SVM, the AT analysis module may augment the data relating to the profile features as described above, such that wider ranges of values in the data point cloud or narrower ranges of values may be considered as lying within the acceptable frontier. Wider ranges of values result in a wider range for data points to be non-outliers, whereas narrower ranges of values result in a narrower range for data points to be non-outliers. Contrary to some machine-learning techniques, the training of the one class SVM is extremely fast, almost real-time. This means that the training of the one class SVM to adapt to a change of conditions in the AT is not detrimental to the execution of the invention. On the contrary, this feature is an advantage of the one class SVM, which is particularly suited to adapt to a potentially quickly varying environment.

The various features may also be combined, such that a data point may be considered an outlier even if its feature values are within their respective expected ranges individually.

Combining the features together allows to detect cases where the overall change is problematic in spite of the fact that the individual feature changes appear regular. For the training of the one class SVM, the most recent input data available may used, that is all data in the memory buffer. Alternatively, a subset thereof may be used.

If the training function 210 was launched after the execution of the idle function 200, the input data collected by the idle function 200 will be used to create the AT profile. If the training function 210 was launched after the detection function 220, the input data from a memory buffer will be used to create a new AT profile, as described below.

In the training function 210, the AT analysis module uses data augmentation techniques to add variability to the data, such that slight offsets from the anticipated profile will not trigger an outlier warning when the AT profile is used in the detection function 220. For example, the data may be augmented by adding a noise tolerance level to the data. After the execution on the training function 210, the AT analysis module executes the detection function 220.

In the detection function 220, the AT analysis module uses the AT profile that was previously built by the training function 210 to detect whether incoming input data are outliers, and whether those outliers indicate a change in the current AT profile.

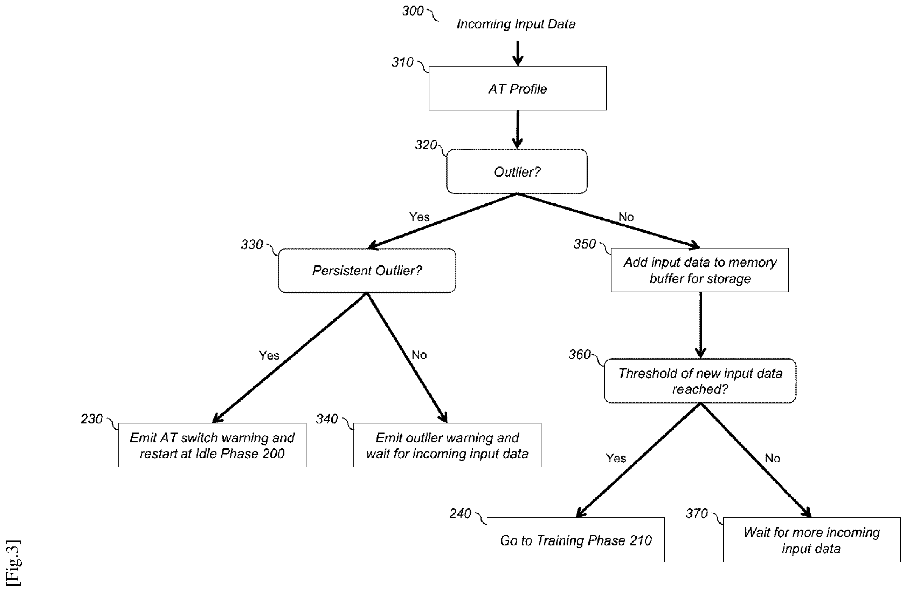

FIG. 3 illustrates an exemplary embodiment of the detection function. In an operation 300, input data is received, and is compared to the current AT profile in an operation 310. Operation 310 determines if the input data of operation 300 is an outlier or not, and the result is tested in an operation 320.

As described above, density estimation techniques are used to determine whether a data point is an outlier. For example, a data point cloud can be used to determine if a data point is an outlier or not. In other words, a data point is an outlier if it is outside the cloud and not an outlier if it is inside the cloud. In the case that a one class SVM is used to determine whether a data point is an outlier, the input data is first transformed in order to extract the features which have been used to train the one class SVM, and these features are thereafter fed to said one class SVM, which returns a value indicative of whether the data point is an outlier or not.

If the input data 300 is an outlier, the AT analysis module proceeds to operation 330, which determines whether the input data 300 is also a persistent outlier. Input data is a persistent outlier if a defined threshold is met. This persistent outlier threshold may be set manually by an EP or other medical professional, or it may be set automatically by the AT analysis module. This persistent outlier threshold may be defined as: a consecutive number of outliers, a percentage of outliers in a sample of input data, a defined standard deviation away from the expected value, or any combination thereof. In a preferred embodiment, the consecutive number of outliers is used.

If input data 300 is an outlier but not a persistent outlier, the AT analysis module outputs a warning in an operation 340 that there was an outlier and waits for more input data.

If input data 300 is an outlier and also a persistent outlier, operation 230 of FIG. 2 and FIG. 3 is executed, such that the AT analysis module outputs a warning that there has been a switch in the AT profile, and the idle function 200 is executed anew with a cleared buffer. An indication is also provided to the EP indicating that there has been a change in the AT profile, such as a visual or auditory signal. This can result in the EP changing strategies or re-starting procedures. For example, the EP could decide to create a new 3D map.

If operation 320 determines that input data 300 is not an outlier, the input data 300 is sent to a memory buffer for storage in an operation 350. The AT analysis module then determines whether a threshold amount of input data in the memory buffer has been reached in an operation 360. This memory buffer threshold may be set manually by an EP or other medical professional, or it may be set automatically by the AT analysis module. This memory buffer threshold also may be set according to: an amount of input data, a time period of collection, or any combination thereof. The memory buffer limit itself can also be used to limit the amount of data stored, such that new incoming data will replace the oldest data saved in the memory buffer.

If the memory buffer threshold has been reached, as shown in operation 240 in FIG. 2 and FIG. 3, the AT analysis module executes the training function 210 anew where the AT analysis module uses the input data in the memory buffer to update the current AT profile. If the memory buffer threshold has not been reached, the AT analysis module waits for more incoming input data in operation 370.

As appears from the above, the combination of the idle function 200, the training function 210 and the detection function 220 guarantee that the AT analysis module constantly analyzes the input data and continuously warn the EP when an AT profile has changed, and updates it under certain conditions.

While the one class SVM provides the best results in the Applicant's tests, it could be replaced in alternative embodiments by another feature-based anomaly detection algorithm such as:
- Isolation forest: Liu, Fei Tony, Ting, Kai Ming and Zhou, Zhi-Hua. "Isolation forest." Data Mining, 2008. ICDM'08. Eighth IEEE International Conference,
- Local Outlier Factor: Breunig, Kriegel, Ng, and Sander (2000) LOF: identifying density-based local outliers. Proc. ACM SIGMOD,
- Robust covariance: Estimating the support of a high-dimensional distribution Schölkopf, Bernhard, et al. Neural computation 13.7 (2001): 1443-1471,
- k-nearest neighbor https://link.springer.com/article/10.1007/s007780050006,
- DBSCAN: Tran Manh Thang; Juntae Kim. "The Anomaly Detection by Using DBSCAN Clustering with Multiple Parameters", 2011 International Conference on Information Science and Applications,
- CBLOF: He, Z.; Xu, X.; Deng, S. (2003). "Discovering cluster-based local outliers". Pattern Recognition Letters,
- Hierarchical density-based cluster analysis: Campello, R. J. G. B.; Moulavi, D.; Zimek, A.; Sander, J. (2015). "Hierarchical Density Estimates for Data Clustering, Visualization, and Outlier Detection". ACM Transactions on Knowledge Discovery from Data,
- Gaussian Mixture: W. Liu, D. Cui, Z. Peng and J. Zhong, "Outlier Detection Algorithm Based on Gaussian Mixture Model," 2019 IEEE International Conference on Power, Intelligent Computing and Systems (ICPICS), 2019, pp. 488-492, doi: 10.1 109/ICPICS4773 1.2019.8942474, or
- Hidden Markov models: A. Sultana, A. Hamou-Lhadj and M. Couture, "An improved Hidden Markov Model for anomaly detection using frequent common patterns," 2012 IEEE International Conference on Communications (ICC), 2012, pp. 1113-1117, doi: 10.1109/ICC.2012.6364527.

## Claims

1. Computer system for analyzing an atrial tachycardia, AT, signal, comprising one or more processors configured to perform the following steps by executing an AT analysis module:
a) receiving a set of data which represent recordings of electrical activity of a human heart from at least one input source;
b) creating a current atrial tachycardia profile using said set of data;
c) receiving a current data point of an atrial tachycardia signal from at least one input source (300); and
d) analyzing the current data point using the current atrial tachycardia profile in order to determine whether said current data point is an outlier with respect to said current atrial tachycardia profile (320),
e) analyzing the current data point using the current atrial tachycardia profile in order to determine whether the current data point is a persistent outlier (330); and
f) emitting a change warning indicating a change in the current atrial tachycardia profile upon determining that the current data point is a persistent outlier (230).

2. Computer system for analyzing an atrial tachycardia signal according to claim 1,
wherein step b) comprises extracting features chosen in a group comprising a cycle length feature, an electrode activation sequence feature, and/or a morphological feature of the activation sequence, and training a feature-based anomaly detection algorithm using at least one of said extracted features and/or a combination thereof.

3. Computer system for analyzing an atrial tachycardia signal according to claim 2,
wherein step b) comprises augmenting the data of the extracted features prior to training said feature-based anomaly detection algorithm.

4. Computer system for analyzing an atrial tachycardia signal according to claim 2 or 3,
wherein said feature-based anomaly detection algorithm is a one class support vector machine.

5. Computer system for analyzing an atrial tachycardia signal according to claim 4,
wherein step d) comprises extracting from said current data point the features which have been used to train said one class support vector machine, feeding said extracted features to said one class support vector machine, and receiving in return a value indicating whether said current data point is an outlier with respect to said current atrial tachycardia profile.

6. Computer system for analyzing an atrial tachycardia signal according to claim 3, comprising:
h) receiving a new set of data which represent recordings of electrical activity of a human heart from at least one input source; and
i) creating a new current atrial tachycardia profile using the second set of data, and repeating steps c) and d) with said new current atrial tachycardia profile.

7. Computer system for analyzing an atrial tachycardia signal according to claim 1,
wherein, when step d) determines that the current data point is not an outlier further comprising
d1) adding the current data point to a memory buffer.

8. Computer system for analyzing an atrial tachycardia signal according to claim 7,
wherein step d) further comprises:
d2) determining that a memory buffer threshold for data has been reached; and
j) creating a new current atrial tachycardia profile using the data stored in the memory buffer and repeating steps c) and d) with said new current atrial tachycardia profile.

9. Computer system for analyzing an atrial tachycardia signal according to claim 7,
wherein step d) further comprises:
d3) determining that a memory buffer threshold for data has not been reached, and repeating steps c) and d) with said current atrial tachycardia profile.

## Patentansprüche

1. Computersystem zur Analyse eines Vorhoftachykardie-Signals (AT), das einen oder mehrere Prozessoren umfasst, die so konfiguriert sind, dass sie durch Ausführung eines AT-Analysemoduls die folgenden Schritte ausführen:
a) Empfangen eines Datensatzes, der Aufzeichnungen der elektrischen Aktivität eines menschlichen Herzens darstellt, von mindestens einer Eingangsquelle;
b) Erstellen eines aktuellen Vorhoftachykardie-Profils unter Verwendung des Datensatzes;
c) Empfangen eines aktuellen Datenpunkts eines Vorhoftachykardie-Signals von mindestens einer Eingangsquelle (300); und
d) Analysieren des aktuellen Datenpunkts unter Verwendung des aktuellen Vorhoftachykardie-Profils, um zu bestimmen, ob der aktuelle Datenpunkt in Bezug auf das aktuelle Vorhoftachykardie-Profil ein Ausreißer ist (320),
e) Analysieren des aktuellen Datenpunkts unter Verwendung des aktuellen Vorhoftachykardie-Profils, um zu bestimmen, ob der aktuelle Datenpunkt ein persistenter Ausreißer ist (330); und
f) Ausgeben einer Änderungswarnung, die eine Änderung des aktuellen Vorhoftachykardie-Profils anzeigt, wenn festgestellt wird, dass der aktuelle Datenpunkt ein persistenter Ausreißer ist (230).

2. Computersystem zur Analyse eines Vorhoftachykardie-Signals nach Anspruch 1,
wobei Schritt b) das Extrahieren von Merkmalen umfasst, die aus einer Gruppe ausgewählt sind, die ein Zykluslängenmerkmal, ein Elektrodenaktivierungssequenzmerkmal und/oder ein morphologisches Merkmal der Aktivierungssequenz umfasst, sowie das Trainieren eines merkmalsbasierten Algorithmus zur Anomalie-Erkennung unter Verwendung mindestens eines der extrahierten Merkmale und/oder einer Kombination davon.

3. Computersystem zur Analyse eines Vorhoftachykardie-Signals nach Anspruch 2,
wobei Schritt b) das Augmentieren der Daten der extrahierten Merkmale vor dem Trainieren des merkmalsbasierten Algorithmus zur Anomalie-Erkennung umfasst.

4. Computersystem zur Analyse eines Vorhoftachykardie-Signals nach Anspruch 2 oder 3,
wobei der merkmalsbasierte Algorithmus zur Anomalie-Erkennung eine Ein-Klassen-Support-Vektor-Maschine ist.

5. Computersystem zur Analyse eines Vorhoftachykardie-Signals nach Anspruch 4,
wobei Schritt d) das Extrahieren der Merkmale aus dem aktuellen Datenpunkt umfasst, die zum Trainieren der Ein-Klassen-Support-Vektor-Maschine verwendet wurden, das Einspeisen der extrahierten Merkmale in die Ein-Klassen-Support-Vektor-Maschine und das Empfangen eines Wertes als Rückmeldung, der angibt, ob der aktuelle Datenpunkt in Bezug auf das aktuelle Vorhoftachykardie-Profil ein Ausreißer ist.

6. Computersystem zur Analyse eines Vorhoftachykardie-Signals nach Anspruch 3, umfassend:
h) Empfangen eines neuen Datensatzes, der Aufzeichnungen der elektrischen Aktivität eines menschlichen Herzens darstellt, von mindestens einer Eingangsquelle; und
i) Erstellen eines neuen aktuellen Vorhoftachykardie-Profils unter Verwendung des zweiten Datensatzes und Wiederholen der Schritte c) und d) mit dem neuen aktuellen Vorhoftachykardie-Profil.

7. Computersystem zur Analyse eines Vorhoftachykardie-Signals nach Anspruch 1,
wobei, wenn in Schritt d) festgestellt wird, dass der aktuelle Datenpunkt kein Ausreißer ist, das Verfahren ferner umfasst:
d1) Hinzufügen des aktuellen Datenpunkts zu einem Speicherpuffer.

8. Computersystem zur Analyse eines Vorhoftachykardie-Signals nach Anspruch 7,
wobei Schritt d) ferner umfasst:
d2) Feststellen, dass ein Speicherpuffer-Schwellenwert für Daten erreicht wurde; und
j) Erstellen eines neuen aktuellen Vorhoftachykardie-Profils unter Verwendung der im Speicherpuffer gespeicherten Daten und Wiederholen der Schritte c) und d) mit dem neuen aktuellen Vorhoftachykardie-Profil.

9. Computersystem zur Analyse eines Vorhoftachykardie-Signals nach Anspruch 7,
wobei Schritt d) ferner umfasst:
d3) Feststellen, dass ein Speicherpuffer-Schwellenwert für Daten nicht erreicht wurde, und Wiederholen der Schritte c) und d) mit dem aktuellen Vorhoftachykardie-Profil.

## Revendications

1. Système informatique pour l'analyse d'un signal de tachycardie auriculaire, AT, comprenant un ou plusieurs processeurs configurés pour réaliser les étapes suivantes en exécutant un module d'analyse AT :
a) la réception d'un jeu de données qui représentent des enregistrements d'activité électrique d'un cœur humain à partir d'au moins une source d'entrée ;
b) la création d'un profil courant de tachycardie auriculaire en utilisant ledit jeu de données ;
c) la réception d'un point de données courant d'un signal de tachycardie auriculaire provenant d'au moins une source d'entrée (300) ; et
d) l'analyse du point de données courant en utilisant le profil de tachycardie auriculaire courant afin de déterminer si ledit point de données courant est une valeur aberrante par rapport audit profil de tachycardie auriculaire courant (320),
e) l'analyse du point de données courant en utilisant le profil de tachycardie auriculaire courant afin de déterminer si le point de données courant est une valeur aberrante persistante (330) ; et
f) l'émission d'une alerte de changement indiquant un changement dans le profil de tachycardie auriculaire courant lorsqu'il est déterminé que le point de données courant est une valeur aberrante persistante (230).

2. Système informatique pour l'analyse d'un signal de tachycardie auriculaire selon la revendication 1,
dans lequel l'étape b) comprend l'extraction de caractéristiques choisies dans un groupe comprenant une caractéristique de longueur de cycle, une caractéristique de séquence d'activation d'électrode et/ou une caractéristique morphologique de la séquence d'activation, et l'entraînement d'un algorithme de détection d'anomalie basé sur des caractéristiques en utilisant au moins une desdites caractéristiques extraites et/ou une combinaison de celles-ci.

3. Système informatique pour l'analyse d'un signal de tachycardie auriculaire selon la revendication 2,
dans lequel l'étape b) comprend l'augmentation des données des caractéristiques extraites avant l'entraînement dudit algorithme de détection d'anomalie basé sur des caractéristiques.

4. Système informatique pour l'analyse d'un signal de tachycardie auriculaire selon la revendication 2 ou 3,
dans lequel ledit algorithme de détection d'anomalie basé sur des caractéristiques est une machine à vecteurs de support à classe unique.

5. Système informatique pour l'analyse d'un signal de tachycardie auriculaire selon la revendication 4,
dans lequel l'étape d) comprend l'extraction à partir dudit point de données courant des caractéristiques qui ont été utilisées pour entraîner ladite machine à vecteurs de support à classe unique, l'alimentation en lesdites caractéristiques extraites de ladite machine à vecteurs de support à classe unique, et la réception en retour d'une valeur indiquant si ledit point de données courant est une valeur aberrante par rapport audit profil de tachycardie auriculaire courant.

6. Système informatique pour l'analyse d'un signal de tachycardie auriculaire selon la revendication 3, comprenant :
h) la réception d'un nouveau jeu de données qui représentent des enregistrements d'activité électrique d'un cœur humain provenant d'au moins une source d'entrée ; et
i) la création d'un nouveau profil de tachycardie auriculaire courant en utilisant le second jeu de données, et la répétition des étapes c) et d) avec ledit nouveau profil de tachycardie auriculaire courant.

7. Système informatique pour l'analyse d'un signal de tachycardie auriculaire selon la revendication 1,
dans lequel, lorsque l'étape d) détermine que le point de données courant n'est pas une valeur aberrante comprenant en outre
d1) l'ajout du point de données courant à une mémoire tampon.

8. Système informatique pour l'analyse d'un signal de tachycardie auriculaire selon la revendication 7,
dans lequel l'étape d) comprend en outre :
d2) la détermination qu'un seuil de mémoire tampon pour les données a été atteint ; et
j) la création d'un nouveau profil de tachycardie auriculaire courant en utilisant les données stockées dans la mémoire tampon et la répétition des étapes c) et d) avec ledit nouveau profil de tachycardie auriculaire courant.

9. Système informatique pour l'analyse d'un signal de tachycardie auriculaire selon la revendication 7,
dans lequel l'étape d) comprend en outre :
d3) la détermination qu'un seuil de mémoire tampon pour les données n'a pas été atteint, et la répétition des étapes c) et d) avec ledit profil de tachycardie auriculaire courant.
